(19) Europäisches Patentamt
European Patent Office
Office européen des brevets

(11) EP 2 859 075 B1

(12) **FASCICULE DE BREVET EUROPEEN**

(45) Date de publication et mention de la délivrance du brevet:
**03.04.2019 Bulletin 2019/14**

(21) Numéro de dépôt: **13737299.1**

(22) Date de dépôt: **24.05.2013**

(51) Int Cl.:
*C11D 1/66* (2006.01) *C11D 3/22* (2006.01)
*C11D 3/43* (2006.01) *A01N 3/00* (2006.01)
*A01N 43/16* (2006.01) *A61K 8/49* (2006.01)
*A61K 31/7028* (2006.01) *A61Q 5/02* (2006.01)
*A61Q 19/00* (2006.01)

(86) Numéro de dépôt international:
**PCT/FR2013/000133**

(87) Numéro de publication internationale:
**WO 2013/182759 (12.12.2013 Gazette 2013/50)**

(54) **BIOSOLUBILISANT**

BIO-LÖSUNGSVERMITTLER

BIOSOLUBILIZER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.06.2012 FR 1201629**

(43) Date de publication de la demande:
**15.04.2015 Bulletin 2015/16**

(73) Titulaire: **Givaudan SA
1214 Vernier (CH)**

(72) Inventeurs:
- **ERNENWEIN, Cédric
F-02860 Nouvion le Vineux (FR)**
- **REYNAUD, Romain
F-51110 Reims (FR)**
- **GUILLERET, Arnaud
F-51110 Boult sur Suippe (FR)**
- **PODEVIN, Lucie
F-62110 Henin-Beaumont (FR)**
- **RANNOU, Alexis
F-02000 Laon (FR)**
- **LAFOSSE, Frédérique
F-51110 Reims (FR)**

(74) Mandataire: **Global Patents
Givaudan International SA
Ueberlandstrasse 138
8600 Dübendorf (CH)**

(56) Documents cités:
**EP-A1- 0 209 783** **WO-A1-2011/051161**
**WO-A2-2004/108063** **FR-A1- 2 779 057**
**JP-A- 2009 275 145** **US-A- 5 756 471**
**US-A- 6 057 302** **US-B1- 6 262 038**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 859 075 B1

## Description

La présente invention concerne l'utilisation d'au moins un sophorolipide comme solubilisant.

La notion de solubilisation a été définie pour la première fois par Mc Bain en 1918 dans "Journal of Chemical society" 113, 825. Selon cette définition, la solubilisation consiste en le passage spontané d'une molécule insoluble dans l'eau (ou partiellement insoluble) dans un solvant donné en formant une solution stable thermodynamiquement. Ici le solvant permettant la solubilisation est une solution aqueuse de tensioactifs. La solubilisation a lieu grâce à la formation d'agrégats de molécules tensioactives, nommés micelles, constituant des nano-domaines non aqueux dans l'eau. La solubilisation peut avoir lieu par incorporation des molécules non solubles dans le coeur des micelles ou par leurs adsorptions sur la surface des micelles ou encore par leur agrégation dans la membrane constituant la paroi de la micelle.

L'usage des biotensioactifs glycolipidiques dans les domaines de la pharmacie, de la cosmétique, de la détergence ou encore de la formulation des produits phytosanitaires est connu. Les glycolipides préférés sont généralement les sophorolipides car, outre leurs efficacités et particularités dans les domaines d'applications cités, ils sont industriellement disponibles et compétitifs.

Les sophorolipides sont constitués d'une partie saccharidique reliée par une liaison glycosidique à un reste d'alcools gras, d'acides gras ou d'esters d'acides gras. La partie saccharidique, composant la partie polaire ou "tête polaire" du tensioactif, est composée d'un reste de sophorose comportant des hydroxyles libres ou acétylés. Les sophoroses sont des sucres particuliers composés de deux unités glucoses reliées en beta 1,2. Les sophorolipides ayant un reste d'acide gras comme partie lipophile, encore nommé "queue lipophile", possèdent au moins une fonction acide carboxylique sous forme acide ou sous la forme dissociée, notamment de sels de métal alcalin. Cette fonction carboxylique peut être estérifiée par un composé comportant au moins une fonction alcool, notamment le méthanol. Pour des radicaux alkyles longs, c'est-à-dire comportant au moins 10 atomes de carbone, et de préférence de 16 à 18 atomes de carbone, une estérification intramoléculaire avec l'un des hydroxyles libres des glucoses de la tête polaire est possible. On parle alors d'une forme lactone. D'une manière générale, les sophorolipides disponibles commercialement sont des mélanges des formes ouvertes acide ou ester, avec des formes lactones ou formes fermées, di-acétylés, mono-acétylés ou non acétylés.

Les sophorolipides font partie de la famille des biotensiocatifs. L'indicatif bio faisant référence à leur mode d'obtention par biotechnologie. Ils sont en effet obtenus par un processus de fermentation. Ce processus à notamment été décrit par Gorin et collaborateurs dans "Canadian Journal of Chemistry", 39, 846 en 1961. Les souches bactériologiques naturelles, sélectionnées ou modifiées capables de métaboliser des sources saccharidiques et des lipides afin de former des glycolipides, et notamment des sophorolipides, sont par exemple *Candida apicola, Candica bombicola, Yarrowia lipolytica, Candida bogoriensis.*

Dans les domaines de la pharmacie et de la cosmétique, l'activité biologique des sophorolipides est particulièrement décrite. L'activité biologique est généralement reliée à une activité bactéricide, bactériostatique, fongicide, virucide, antimicrobienne ou spermicide. WO 2006/069175 A2 décrit, par exemple, une activité antifongique, WO 2005/089522 une activité spermicide et virucide, WO 2007/130738 une action pour lutter contre l'herpès. Pour ces domaines d'applications, les formes lactones sont généralement isolées, notamment par des procédés de chromatographie, lourds et coûteux. Certains nécessitent des fonctionnalisations par voie chimique, notamment des estérifications, et acétylation des hydroxyles de la tête polaire pour exercer leurs activités biologiques.

Dans le domaine plus spécifique de la cosmétique, les sophorolipides sont utilisés pour des applications sur la peau ou les cheveux. WO 97/01343 traite, par exemple, d'une application liée à la dépigmentation de la peau, WO 2004/108063 de la régulation de la masse adipeuse, EP 0209783 de l'activité antipelliculaire.

FR 2 779 057 A1 (INSTITUT FRANÇAIS DU PETROLE [FR]), US 6 057 302 A (BORZEIX FREDERIQUE [FR]), US 5 756 471 A (HILLION GERARD [FR] ET AL), US 6 262 038 B1 (PIERCE DEBORAH [US] ET AL) et JP 2009 275145 A (SARAYA KK) décrivent une composition comprenant un sophorolipide, un composé insoluble dans l'eau et de l'eau.

Les sophorolipides, sous des formes isolées, purifiées ou modifiées chimiquement, sont donc utilisés comme des molécules ayant une activité biologique ciblée. Ils sont alors formulés comme tels, avec des adjuvants de formulations, notamment des émulsionnants et d'autres tensioactifs, dans des préparations dermatologiques ou capillaires, sous la forme d'émulsions huile dans eau (H/E) ou eau dans huile (E/H) ou des solutions.

L'utilisation de formes brutes des sophorolipides, c'est-à-dire issus directement du processus de fermentation, ou extraits du milieu de fermentation, en tant que solubilisant de composés non complètement solubles dans l'eau n'a jamais été décrite. Par non complètement solubles dans l'eau, on entend des composés non solubles dans l'eau dans une gamme de température variant de 0 à 100°C, ou partiellement solubles, c'est-à-dire que la zone de solubilité du composé dans l'eau est limitée en concentration et/ou en température et/ou en force ionique de l'eau de dilution. S'entendent donc comme non complètement solubles des molécules organiques, mais aussi des tensioactifs non ioniques possédant des points de troubles inférieurs à 100°C, et des tensioactifs ioniques possédant des points de Krafft ou de cristallisation supérieurs à 0°C.

Dans le domaine de la détergence, l'utilisation des glycolipides, et plus particulièrement des sophorolipides, se réfère à des propriétés tensioactives, notamment pour des activités de nettoyage du linge et des surfaces dures. Le document EP B0499434 traite de l'efficacité de compositions contenant des sophorolipides en mélange avec d'autres tensioactifs anioniques ou non ioniques pour le nettoyage du linge. EP 1411111 A1 traite de compositions faiblement moussantes contenant des sophorolipides, notamment des mélanges des formes lactones et des formes ouvertes. EP 1445302 A2 concerne des compositions pour le nettoyage des surfaces dures, notamment des surfaces en polycarbonate, contenant des glycolipides en mélange avec des tensioactifs ne formant pas de phases lamellaires. Dans tous ces exemples, les propriétés mises en évidence sont liées à des effets de synergie des mélanges de tensioactifs contenant au moins des sophorolipides pour le nettoyage ou encore à la faible production de mousse des sophorolipides en solution.

La solubilisation de composés non complètement solubles à l'aide des sophorolipides combinée au renforcement de l'action de nettoyage n'est jamais décrite. En particulier, la solubilisation des solvants peu ou non polaires par des sophorolipides afin d'augmenter l'action de nettoyage, plus particulièrement de dégraissage, des surfaces dures n'a jamais été démontrée.

La demande de brevet WO 2011/051161 A1 décrit des compositions à base de sophorolipides et de solvants. La demande de brevet porte sur la formulation d'agent de nettoyage des surfaces dures permettant de laisser peu de traces après séchage, notamment sur les vitres. Les sophorolipides sont ici utilisés uniquement avec des solvants polaires solubles dans l'eau, notamment des alcools et des éthers de glycols. Dans la description de l'invention et les exemples, les auteurs prétendent qu'il est nécessaire d'ajouter des solubilisants et/ou des hydrotropes, notamment le xylènesulfonate de sodium et/ou de l'éthanol pour stabiliser les solutions.

Description de l'invention :

La présente invention concerne les capacités de solubilisation des sophorolipides et l'utilisation des sophorolipides comme agent de solubilisation dans des préparations cosmétiques, de soins des cheveux et de la peau, des préparations pour le nettoyage du linge et des surfaces dures, ainsi que dans des préparations pour le traitement des plantes.

Des compositions pour le nettoyage des surfaces dures contenant au moins un sophorolipide, un solvant non aqueux non polaire et de l'eau sont aussi décrites. Ces compositions montrent des capacités de dégraissage remarquables grâce à l'action combinée des sophorolipides et des solvants. De plus, elles ne contiennent pas nécessairement des alcools volatils et inflammables, notamment pas d'éthanol, ni d'éthers de glycols, ce qui représente une avancée technique indéniable.

L'un des avantages des solubilisants est qu'ils permettent l'obtention de solutions parfaitement limpides, ce même en présence de molécules non solubles. D'une manière générale la solution est composée en majorité d'eau et ladite substance non soluble donnerait une solution turbide ou une phase distincte supplémentaire sans l'ajout du solubilisant. La limpidité de la solution en présence du solubilisant et de ladite substance non soluble peut être déterminée de différentes manières, notamment à l'aide d'un turbidimètre muni d'une source lumineuse à 860 nm ou d'un spectromètre permettant une mesure de l'absorbance à l'aide d'une source lumineuse à 600 nm. Dans les deux cas, la limpidité sera assurée si l'absorbance est comprise entre 0 et 0,1, de préference entre 0 et 0,05 ou si la valeur NTU (Unité de Turbidité Néphélométrique) est inférieur à 20, de préference inférieur à 10. Une autre méthode consiste à mettre 20g de liquide à évaluer dans un flacon en verre de 30 ml, à placer derrière le flacon une feuille blanche sur laquelle sont dessinés des carrés noir de 2 cm de côté (formant ainsi un quadrillage noir et blanc). La limpidité sera jugée visuellement en plaçant un flacon contenant de l'eau filtrée (0,45$\mu$m) à côté du flacon contenant la solution à évaluer. La solution sera limpide si la netteté des carreaux noir et blanc est équivalente à celle pour de l'eau filtrée.

La méthode préférée pour obtenir une solution limpide consiste dans une première étape à ajouter la substance non soluble à une solution aqueuse concentrée contenant de 100 à 10 % en poids, de préférence de 100 à 50 % du solubilisant, en agitant le mélange à une température comprise entre 10 et 100°C, de préférence entre 20 et 60°C, de préférence encore à la température ambiante. Le mélange obtenu est maintenu sous agitation, par exemple à l'aide d'un moteur et d'une pâle d'agitation, par exemple encore à l'aide d'un barreau magnétique et d'un aimant rotatif placé sous le flacon, par exemple encore à l'aide d'ultrasons, d'un agitateur de type rotor-stator, d'un moulin colloïdal, par agitation manuelle. La vitesse d'agitation pourra varier de 1 à 50000 tours par minute, de préférence de 1 à 100 tours par minute. La durée d'agitation sera généralement comprise entre 0,05 et 300 minutes, de préférence entre 1 et 10 minutes. Après cette étape de mélange intime entre le solubilisant et la substance à solubiliser, les autres constituants sont ensuite ajoutés sur ce pré-mélange tout en maintenant l'agitation. De manière préférée, la quantité d'eau nécessaire ou la partie d'eau restante sera ajoutée en dernier afin d'obtenir une solution limpide telle que définie précédemment.

Le rapport de masse entre le solubilisant et la matière solubilisée varie de 1 à 9000 et de préférence de 1,6 à 90, et l'eau compose de 1 à 99,89 % de la masse totale de la matière solubilisée, du solubilisant et de l'eau.

La performance de solubilisation peut être exprimée soit de manière empirique, soit en utilisant des outils

prédictifs. Parmi ces outils, les paramètres de Hansen sont vraisemblablement les plus usités. La théorie de Hansen reprend le principe de paramètre de solubilité globale de Hildebrand, qui est défini comme la racine carrée de la densité d'énergie de cohésion par unité de volume. L'énergie de cohésion étant reliée à l'enthalpie de vaporisation selon les principes de thermodynamiques est une grandeur mesurable. Néanmoins le paramètre de solubilité globale apparaît insuffisant pour décrire les phénomènes de solubilité, notamment les interactions entre molécules. Les paramètres de Hansen, décomposent le paramètre global de solubilité de Hildebrand, en composante dispersif ou δd, polaire ou δp et force de liaison hydrogène ou δh (équation 1). Ils sont décrits dans le livre"Hansen Solubility Parameters : A user's handbook" de Charles M. Hansen aux éditions CRC Press, et peuvent être calculés à l'aide de logiciels selon la méthode de contribution de groupes, notamment selon la méthode de Van Kervelen ("Properties of Polymers" aux éditions Elsevier, en 1990. Le $MPa^{1/2}$ est l'unité SI la plus répandue, mais il existe une ancienne unité, en $Cal^{1/2}\ cm^{-3/2}$, encore utilisée quelquefois. Pour transformer cette ancienne unité en unité SI, il suffit de multiplier le paramètre de solubilité correspondant par 2,0455.

$$\delta^2 = \delta_d^2 + \delta_p^2 + \delta_h^2 \qquad \text{Equation 1}$$

Par solvants non aqueux non polaires, utilisés dans les compositions décrites, s'entendent des solvants choisis parmi ceux montrant au moins une zone de non solubilité dans l'eau et caractérisés par un paramètre de Hansen polaire ou δp inférieur à 5,5 $Mpa^{1/2}$, de préférence inférieur à 5,1 $Mpa^{1/2}$, de préférence encore inférieur à 4,1 $Mpa^{1/2}$.
Une autre caractéristique particulière de l'invention concerne la solubilisation d'actifs non solubles, de parfums, d'huiles essentielles, de colorants, de pigments non solubles dans l'eau dans des préparations contenant au moins un sophorolipide et de l'eau. On a trouvé que les sophorolipides pouvaient être utilisés comme des solubilisants, notamment pour les secteurs de la cosmétique et la pharmacie. Dans ces secteurs d'activités, les solubilisants les plus utilisés contiennent au moins un dérivé polyoxyéthyléné, très couramment une huile de ricin polyéthoxylée à 40 motifs d'oxyde d'éthylène, comme le Cremophor CO40 de BASF. Les substituts à ces solubilisants polyéthoxylés, justifiés par la recherche d'ingrédients n'utilisant pas d'oxyde d'éthylène ou de propylène, n'ont généralement pas un niveau d'efficacité équivalent. L'homme du métier à souvent recours à des mélanges de tensioactifs et solvants afin d'augmenter les capacités de solubilisation face à l'éventail des molécules à solubiliser. Les sophorolipides ont des capacités de solubilisation en phase aqueuse remarquables, sans qu'il soit nécessaire d'ajouter d'autres tensioactifs ou solvants. De plus, ils ne contiennent pas de motif oxyde d'éthylène ou de propylène, sont issus uniquement de matières renouvelables et sont fabriqués selon un processus de fermentation moins énergivore que les processus de la chimie classique. A ce titre, les sophorolipides permettent de combler ce manque de solubilisant d'origine végétale et plus particulièrement de biosolubilisant.

Les documents WO2004/108063, FR2779057, US6057302, US5756471, EP0209783, WO2011/051161, US6262038 et JP2009275145 ne décrivent pas de composition limpide.

Les sophorolipides utilisés dans la présente invention possèdent les structures générales suivantes :

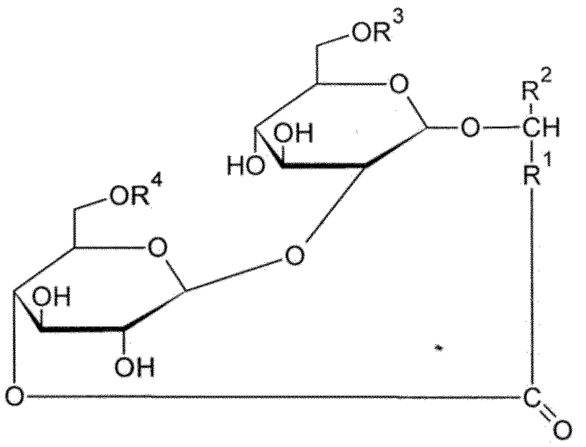

(1)

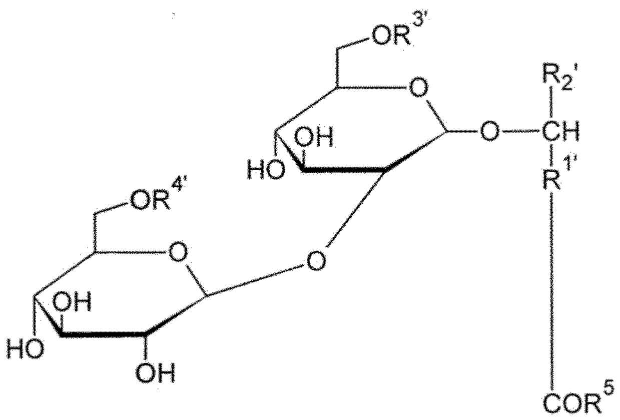

(2)

R1 et R1' sont indépendamment les unes des autres des chaînes hydrocarbonées saturées ou ayant une ou plusieurs insaturations, non hydroxylées ou possédant un ou plusieurs groupements hydroxyles, linéaires ou ramifiées ayant 1 à 21 atomes de carbone, notamment dérivées d'un radical alkyl.

R2 et R2' sont indépendamment les uns des autres un atome d'hydrogène ou un radical alkyle saturé ou ayant une ou plusieurs insaturations, non hydroxylé ou possédant un ou plusieurs groupements hydroxyles, linéaires ou ramifiés ayant 1 à 9 atomes de carbone.

R3, R4, R3' et R4' sont indépendamment les uns des autres un atome d'hydrogène ou un groupement acétyle.

R5 est un groupement $OCH_3$ ou OH ou $O^-M^+$ où M+ est un ion métallique ou un cation organique, notamment des sels d'ammonium comme de diméthylammonium, triméthylammonium, isopropylammonium, mono-éthanol-ammonium, di-éthanol-ammonium, tri-éthanol-ammonium, dis phosphonium ou $O(CH_2)_mCH_3$ avec m compris entre 1 et 11.

Les composés (1) et (2) constituent l'un et l'autre ou l'un ou l'autre le sophorolipide utilisé dans l'invention.

Les sophorolipides industriellement disponibles comprennent, en plus des composés (1) et/ou (2), des impuretés. Les impuretés peuvent être des alcools gras, des esters d'acides gras, des triglycérides ou huiles, des sucres notamment des glucoses, des sophoroses, des acides organiques sous leurs formes acides ou dissociés, notamment des acides gras, de l'acide acétique,

Les molécules pouvant être solubilisées en phase aqueuse par les sophorolipides dans des préparations utilisées dans l'invention peuvent être des actifs, c'est-à-dire des molécules ayant une action ciblée, biologique ou chimique.

Les sophorolipides seront donc des adjuvants technologiques indispensables permettant, en particulier, la formulation de solutions, lotions, gels aqueux, de microémulsions contenant un ou plusieurs actifs solubilisés.

Au titre des actifs, citons par exemple, sans l'intention de s'y limiter:

- les vitamines, comme la vitamine A, E ou C,
- les anti-inflammatoires, comme des extraits de plantes, l'alpha-bisabolol, le panthénol, l'alpha-tocophérol,
- les anti-brûlures, comme l'allantoïne,
- les anti-âges, comme le rétinol,
- les agents pigmentant la peau et/ou des agents dépigmentant la peau, comme l'acide kojique, l'acide coumarique, l'arbutine,
- les actifs amincissants, comme la caféine, les phytostérols, la phloridizine, des extraits de quinoa,
- les agents anti-transpirants, comme des sels d'aluminium, de zinc ou de zirconium, l'acide pentaacétique de diéthylène triamine,
- les agents antipelliculaires, comme la pyrithione de zinc ou d'aluminium, l'acide salicylique, des sels de pyridone et des dérivés des pipérazine,
- les filtres solaires organiques dans l'UV-A et/ou l'UV-B pour protéger la peau ou les cheveux des agressions du soleil et des rayons UV, comme les composés autorisés dans la directive européennes N°76/768/CEE, ses annexes et les modifications ultérieures, et notamment les dérivés de la benzophénone, les esters d'acides cinnamiques, les esters d'acide salicylique, le 3-benzylidène camphre,
- les antioxydants, comme l'acide ascorbique et ses dérivés, l'acide citrique et ses dérivés, l'acide glutamique, les glutamates et ses dérivés, l'acide lactique et ses dérivés, l'acide tartrique et ses dérivés, les bioflavonoides, le buthylhydroxy-hydroxyanisol, le carotène et ses dérivés, les sulfites comme le bisulfites de sodium, le chlorobutanol,
- les agents conservateurs, comme les parabens, le phénoxyéthanol, les formaldéhydes, le pantanediol, l'acide sorbique,
- les agents répulsifs pour les insectes comme l'acétamipirid, l'etofenprox, le permethrin, la cypermethrin, le N,N-diéthyl-m-toluamide, l'acétyl-aminopropionate de butyle,

- les actifs pharmaceutiques, comme les désinfectants tels que les dérivés de la chlorexidrine, de l'acide benzoïque, les anti-inflammatoires, comme la teinture d'arnica, l'eucalyptol, le menthol, le diméthoxy-1,2-benzène, les antiacnéiques, comme les dérivés de la trétinoïde, l'acide azelaïque, l'acide salicylique, les agents antibrûlures, comme les dérivés de triéthanolamine, les antifongiques, comme les dérivés de la pyridone, tels que le cyclopiroxolamine, les dérivés d'imidazole, tels que le clotrimazole, l'acide folique, la riboflavine,
- les pigments naturels ou synthétiques non solubles dans l'eau,
- les arômes naturels ou de synthèses non solubles dans l'eau,
- les matières actives phytosanitaires, comme les herbicides, les fongicides, les insecticides tels que ceux décrits dans "The Pesticide Manual" (9e édition, C.R. Worlkling et R.J Hance, éditeurs, publié par the British Crop Protection Council) sous leurs formes non solubles dans l'eau.

Dans l'utilisation selon l'invention, la matière non soluble peut être un composé parfumé ou une huile essentielle. Les composés odorants non solubles dans l'eau sont mis en contact avec une solution contenant au moins un sophorolipide permettant leur complète solubilisation dans la solution contenant de l'eau. Au titre des parfums et des huiles essentielles, citons, sans l'intention de s'y limiter:

- les parfums synthétiques ou naturels, comme l'acétate de benzyle, l'acétate de linalyle, de benzyle, de terpényle, de vetyvéryle, d'amyle, de bornyle, de cédryle, de géranyle, de phényléthyle, de paracrésyle, de styrallyle, les butyrates d'amyle, le benzoate de linalyle, le citral, le citronellal, le lilial, l'eugénol, le géaniol, citronelol, le linalool et les autres dérivés terpéniques, l'alcool anisique, l'alcool cinnamique, l'alcool styrallique, les aldéhydes tels que les aldéhydes octylique, nonylique, décylique, undécylénique, laurique, myristique, cétylique, stéarique, benzoïque, anisique, le camphre synthétique, le limonène,
- les essences de sauge, de camomille, d'oeillet, de vétiver, de lavandin,
- les huiles essentielles, comme les huiles essentielles de lavande, thym, sarriette, sauge, menthe, cumin, carvi, anis vert, fenouil, aneth, eucalyptus, cajeput, niaouli, girofle, pin, cèdre, cyprès, genévrier, citron, orange, bergamote, cannelle, laurier, camomille.

Une autre utilisation décrite concerne la formulation de produit pour le nettoyage des surfaces dures et des tissus.
Outre la présence d'actifs non solubles dans l'eau, de composés parfumés ou des huiles essentielles non solubles dans l'eau tels que ceux décrits plus haut, les compostions peuvent contenir des solvants non polaires non solubles dans l'eau.
Les sophorolipides sont ajoutés à la composition de nettoyage à la fois pour de bonnes propriétés tensioactives et aussi pour leur capacité de solubilisation. Les solvants sont eux ajoutés afin d'augmenter la performance de nettoyage, notamment du dégraissage. La formulation de solutions, de lotions, de gels aqueux, de microémulsions contenant au moins un solvant non polaire non aqueux étant préférée à d'autres formulations, notamment aux émulsions huile dans eau, car elles sont plus stables dans le temps.
Les solvants non polaires sont ceux dont le paramètre de solubilité polaire de Hansen (noté dp) est inférieur à 5.5 $MPa^{1/2}$, de préférence inférieur à 5,1 $MPa^{1/2}$, et de préférence encore inférieur à 4,1$MPa^{1/2}$.
Citons dans cette catégorie sans l'intention de s'y limiter:

- les alcanes, notamment l'heptane, l'hexane, le pentane, l'octane, le décane, le dodécane, l'hexadécane, et autres alcanes ayant des paramètres de solubilité polaire de Hansen égal à 0,
- les huiles paraffiniques désaromatisées ou partiellement désaromatisées, comme les distillats légers de pétrole, le White Spirit, les hydrocarbures linéaires et/ ou ramifiés de C8 à C18, les hydrocarbures cycliques, ces solvants ayant un paramètre de solubilité polaire de Hansen égal à 0 ou inférieur à 1,
- des hydrocarbures aromatiques, notamment les huiles naphta légères (dp=0.7), le naphtalène (dp=2.0), le benzène (dp=0), le toluène (dp=1,4), l'éthyl-benzène (dp=0,6), le o-xylène (dp=1), le toluène (dp=1,4), le o-n-butyltoluène (dp=0.1),
- des dérivés chlorés, notamment le chloroforme (dp=3.1), le 1,1,2 trichloroéthylène (dp=3.1),
- les huiles végétales ou animales, notamment le saindoux, le suif, l'huile d'arachide, l'huile de beurre, l'huile de graine de coton, l'huile de lin, l'huile d'olive, l'huile de palme, l'huile de pépins de raisin, l'huile de poisson, l'huile de soja, l'huile de ricin, l'huile de colza, l'huile de coprah, l'huile de noix de coco, l'huile de sésame, l'huile de pin, les huiles végétales ayant généralement des paramètres de solubilités polaires de Hansen compris entre 3 et 4,
- les triglycérides de chaînes moyennes, notamment le triglycéride en C8/C10 ou MCT (dp=3.6),
- les esters d'huiles végétales, notamment l'ester méthylique de colza, l'ester méthylique de tournesol, l'oléate de méthyle, le myristate d'isopropyle, le laurate d'amyle, le stéarate d'isostéaryle, les esters d'huiles végétales ayant des paramètres de solubilités polaires de Hansen généralement inférieurs à 2,
- des acides gras issus d'huiles végétales, notamment l'acide oléique (dp=3.1), l'acide octanoique (dp=3.3),

- des acétates, notamment l'acétate de butyle (dp=3.7), l'acétate d'isobutyle (dp=3.7), l'acétate d'amyle (dp=3.3), l'acétate d'isopropyle (dp=3.3),
- des terpènes, notamment le limonène (dp=1,8), le p-cymène (dp=0,6), l'alpha-pinène (dp=4.3), le farnésène, le farnésol (dp=3.8),
- des alcools, notamment l'alcool oléique(dp=2.6), le 2-éthyl-hexanol (dp=3.3), l'octanol (dp=3.3), le nonanol, l'isodécanol, le décanol (dp=2.7)l, le dodédanol, le cyclohexanol (dp=4.1), les alcools amyliques (dp=4.7), le tridecanol (dp=3.1), l'hexyl-décanol, le butyl-octanol,
- des carbonates, notamment le carbonate de diméthyle (dp=3.9), le carbonate de di-éthyle (dp=3.1), le carbonate de di-n-propyle (4,1), le carbonate de 1,2-dodecane (dp=2.6),
- des esters d'acides organiques, notamment le succinate de di-éthyle (dp=4,1), le succinate de dibutyle (dp=2.9), le succinate de diamyle (dp=3.7), le succinate de di-isooctyle (dp=2.7), le succinate de di-octyle (dp=2.7), le succinate de di-décyle (dp=2.3), les esters dibasiques (DBE Esters de la sociétés Invista) (dp de 4,3 à 5.1), le DBE-IB de Invista (dp=2.6), le Rhodiasolv RPDE, IRIS DIB de la société Rhodia,
- des amines, notamment la triméthyl amine(dp=3.4), la 3-méthoxy-propylamine (dp=3.9), la butylamine (dp=4.5) la cyclohexylamine (dp=3.1), la di-éthylamine (dp=2.25), la di-allyl-amine (dp=4.5) l'octylamine, la décylamine, l'oléylamine,
- des éthers, notamment le méthylal (dp=1.8), le triéthylène glycol mono-oléyl éther (dp=3,1), le Méthyl-t-butyl éther (dp=3,5).

Seront exclues de la présente invention les compositions contenant au moins un sophorolipide et un solvant polaire et soluble dans l'eau, notamment le méthanol, l'éthanol, l'isopropanol, l'éthylène glycol diéthyl éther, l'éthylène glycol dibutyl éther, l'éthylène glycol butyl éthyl éther, l'éthylène glycol mono-isopropyl éther, le propylène glycol monobutyl éther, le propylène glycol monopropyl éther. En particulier seront exclus de la présente invention les solvants décrits dans le document WO 2011/051161 A1.

Une composition comprenant en poids de 0,1 à 90%, de préférence de 0,1 à 35%, de préférence encore de 1 à 15%, de sophorolipides et de 0,01 à 65%, de préférence de 0,1 à 35%, et de préférence encore de 0,1 à 5%, d'une matière ou mélange de matières non complètement solubles dans l'eau, le reste étant composé d'une solution aqueuse, est aussi décrite. Cette solution aqueuse composant le reste de la composition décrite contient au moins de l'eau, et éventuellement des adjuvants de formulations solubles dans l'eau, notamment des tensioactifs non ioniques, anioniques, cationiques, des sels, des ajusteurs de pH, des agents hydratants, des chélatants, des ions métalliques, des polymères, des agents dispersants, des colorants, des conservateurs, des hydrotropes.

Au titre des tensioactifs non ioniques, citons, sans l'intention de s'y limiter: les polyglycosides d'alkyle, notamment les C8/C10 polyglucosides, le C8/C10 wheat bran glycoside sous le nom commercial Appyclean 6781 de la société Wheatoleo, les C12/C14 polyglucosides, aussi les alcools gras polyéthoxylés notamment l'alcool C12/C15 à 7 motifs éthoxylés, le C9/C11 à 4 motifs éthoxylés, le C9/C18 à 5 motifs éthoxylés, aussi acides gras éthoxylés comme les acides oléiques éthoxylés, aussi les esters de sorbitan éthoxylé comme le laurate de sorbitan à 20 motifs éthoxylés, aussi les triglycérides éthoxylés comme le saindoux, le suif, l'huile d'arachide, l'huile de beurre, l'huile de graine de coton, l'huile de lin, l'huile d'olive, l'huile de palme, l'huile de pépins de raisin, l'huile de poisson, l'huile de soja, l'huile de ricin, l'huile de colza, l'huile de coprah, l'huile de noix de coco) poly-éthoxylés, aussi les alkylpoly-glucosamides, aussi les glucamides, aussi les oxydes d'amine tels que les oxydes d'alkyl C10/C18 diméthylamines, les oxydes d'alkoxy C8/C22 éthyldihydroxyéthylamines, aussi les esters de polyglycérol éthoxylés tels que le glycéreth 17 cocoate, le glycéreth 7 caprylate caprate, le glycéreth 20 stéarate, aussi les amines éthoxylées, aussi les amides grasses éthoxylés, aussi les copolymères d'oxydes de propylène et d'oxydes d'éthylène.

Au titre des tensioactifs anioniques, citons, sans l'intention de s'y limiter, les alkylbenzènesulfonates, de préférence linéaires, tels que n alkyl en C10/C12 benzène sulfonate de sodium, aussi les paraffine-sulfonates de formule R-CH(SO3M)-R', où R et R' sont des restes de paraffine et M un cation de métal alcalin, aussi les esters d'alkyl sulfonate de formule R-CH(SO3M)-COOR', où R est un radical alkyle de C8 à C20 et R' un radical alkyle de C1 à C6, M un cation de métal alcalin, aussi des alkylsulfates de formule ROSO3M, où R est un radical alkyle ou hydroxyalkyle de C8 à C24 et M est cation de métal alcalin, aussi les alkyléthersulfates de formule R-O-(CH2-CH2O)n-SO3M, où R et M sont définis comme précédemment et n représente le nombre de motifs d'oxyde d'éthylène et est généralement compris entre 0,5 et 6, de préférence entre 2 et 3, aussi les sulfosuccinates de formule R-O-CO-SH(SO3M)-CH2-COOH, où R et M sont définis comme précédemment, de préférence R est compris entre 12 et 14, aussi les alkylamides sulfates de formule RCONHR'OSO3M, où R représente un radical alkyle de C2 à C22, R' un radical alkyle de C2 à C3 et M un cation de métal alcalin, aussi leurs dérivés polyéthoxylés comportant de 0,5 à 60 motifs éthoxylés, aussi les savons d'acides gras saturés ou insaturés de C8 à C16, aussi les alkylglycérol sulfonates, aussi les N-acyl-N-alkyltaurates, les alkylphosphates, aussi les carboxylates de polyoxyéthylènes, aussi les alkyliséthionates, aussi les alkylsuccinamates, aussi les N-acyl-sarcosinates, aussi les sulfates d'alkylglycosides.

Au titre des tensioactifs cationiques, citons, sans l'intention de s'y limiter, des halogénures d'alkyltriméthylammonium, aussi des halogénures de dialkyldiméthylammonium, aussi des dialkylimidazolines quaternaires, aussi des dialkylamidoamines quaternaires, aussi des esters de dialkylamidazoline, aussi des chlorures de dialkyesters de dihydroxypropylammonium quaternaires, aussi des méthylsulfates de dialkylester de triéthanolammonium quaternaires.

Au titre des tensioactifs amphotères, citons, sans l'intention de s'y limiter, les alkyldiméthylbétaines, aussi les alkylamidopropyldiméthylbétaines, aussi les alkyltriméthylsulfobétaines, aussi les produits de condensation d'acides gras et d'hydrolysats de protéines.

Au titre des ingrédients permettant d'ajuster le pH de la solution, citons, sans l'intention de s'y limiter, pour obtenir des pH alcalins, la soude, la potasse, l'ammoniaque, la monoéthanol amine, la tri-éthanolamine, le tripolyphosphate de sodium, le carbonate de sodium, le bicarbonate de sodium, les silicates, notamment le métasilicate de sodium, le gluconate de sodium, l'hydrogénophosphate de sodium. Pour obtenir des pH acides, citons par exemple l'acide sulfurique, l'acide chlorhydrique, l'acide phosphorique, l'acide lactique, l'acide citrique, l'acide glycolique, acide sulfamique, l'acide acétique, l'acide formique, l'acide oxalique.

Au titre des complexants, citons, sans l'intention de s'y limiter, l'EDTA, le NTA, le citrate de sodium, l'IDS (l'Iminodisuccinate tel que le baypure CX100 de la société Lanxess), le MGDA (l'acide méthylglycine-diacétique tel que le Trilon M de BASF), le DTPA (l'acide diéthylènetriaminepentaacétique tel que le trilon C de BASF), le HEDTA (l'acide hydroxyéthyléthylènediamine-triacétique tel que le Trilon D de BASF), l'HEIDA (l'acide N-(2-hydroxyéthyliminodiacétique).

Au titre des sels, citons, sans l'intention de s'y limiter, le chlorure de sodium, le chlorure de potassium.

Au titre des agents hydratants, citons, sans l'intention de s'y limiter, le glycérol, les polysaccharides tels que ceux d'acide hyaluronique.

Au titre des agents épaississants et viscosants, citons, sans l'intention de s'y limiter, les polymères naturels, comme les gommes de guar, les gommes de xanthanes, l'agar-agar, les carraghénanes, les alginates, les pectines, les amidons natifs ou modifiés, les dérivés de celluloses, comme l'hydroxy-éthyle-cellulose, les gélatines d'origine animale, aussi les polymères synthétiques, comme les polymères acryliques, les polymères vinyliques, comme les alcools polyvinyliques aussi les argiles.

Au titre des hydrotropes, citons, sans l'intention de s'y limiter, les xylènes sulfonate de sodium, le p-toluène sulfonate de sodium, le cumène sulfonate de sodium, le 2-éthyl-hexyle sulfonate de sodium, les polyglucoside d'alkyle de formule R-O-(G)n, où R est un groupement alkyle linéaire ou ramifié comportant de 4 à 8 atomes de carbone, G est un reste de glucose, n représente le degrés d'holigomérisation et est un nombre compris entre 1 et 3, l'amyl-xylsodise, des mono-alkyl d'isosorbide, l'urée, le butyl-monoglycol sulfate de sodium.

**UTILISATION**

Les compositions décrites seront particulièrement efficaces pour le nettoyage des surfaces dures. Elle seront donc notamment utiles pour la fabrication de produits multi-usages ménagers, de sprays dégraissants pour les surfaces de la cuisine, de nettoyants pour le four et les plaques de cuisson, les produits de lavage de la vaisselle à la main ou en machine, les produits pour le nettoyage des sols effectués manuellement ou mécaniquement, les produits pour la salle de bains, et notamment les sprays pour le lavage de la baignoire et des lavabos, des gels pour le nettoyage des WC, des sprays pour le nettoyage des vitres et des miroirs, des lingettes pour le nettoyage des surfaces.

Les compositions décrites pourront aussi être utiles à la formulation de produits pour les textiles, notamment des lessives liquides, des gels ou des liquides détachants, des adoucissants, des produits pour la moquette, des produits pour l'intérieur des véhicules.

Pour le nettoyage les compositions décrites seront utilisées dans les domaines ménagers, mais aussi institutionnels, notamment pour la restauration collective et l'hôtellerie, le nettoyage des sols et des mobiliers dans les bureaux et les entreprises, mais aussi industrielles, notamment pour le nettoyage en place dans le domaine agro-industriel, de la cosmétique, des encres, des lubrifiants, du traitement des métaux, le dégraissage des pièces métalliques ou non métalliques, le nettoyage des véhicules, des avions.

Pour le nettoyage des surfaces dures, et plus particulièrement le dégraissage, la composition décrite et contenant au moins un sophorolipide et un solvant non soluble dans l'eau non polaire sera ajustée à un pH compris entre 3 et 12, de préférence entre 4,5 et 9,5 et de préférence encore de 5 à 7,5.

Le pH natif des sophorolipides étant de manière générale acide, généralement d'une valeur de pH comprise entre 3 et 5 à la concentration de 5 à 55% dans l'eau. Pour abaisser cette valeur de pH, il conviendra d'ajouter à la solution contenant les sophorolipides un acide, de préférence un acide moyennement fort ou faible tel que l'acide citrique, lactique, glycolique, propanoïque, acétique, butyrique, formique, valérique, benzoïque, salicylique.

Pour augmenter cette valeur de pH, il conviendra d'ajouter à la solution contenant les sophorolipides une base, de préférence une base faible ou moyennement forte, telle que la monoéthanolamine, la diéthanolamine, la triethanolamine, la triéthylamine, la diéthyliso-propylamine, l'ammoniac, le trisodium citrate, le glutamate de sodium, les polyphosphates, le pyrophosphate de tetrapotassium (ou TKPP), le bicarbonate de sodium, les phosphates tels que l'hydrogénophophate de sodium ou de potassium, les silicates tels que le metasilicate de sodium.

Dans certain cas, notamment pour le nettoyage industriel, il conviendra d'augmenter très fortement le pH des solutions, généralement à des valeurs de pH supérieur à 10. Dans ce cas, l'usage de base forte sera préconisée, tel que la soude, l'hydroxyde de potassium, les hydroxydes de calcium.

Pour le nettoyage ménager, les sprays de dégraissage seront de préférence ajustés au pH compris entre 5,5 et 7, ce qui permet de proposer des produits efficaces et compatibles avec la peau et les muqueuses.

Pour leurs propriétés de solubilisation des actifs, des huiles essentielles et des parfums dans les compositions décrites seront aussi utilisés pour la fabrication de produits de soins de la personne. Notamment, pour la fabrication de gels pour la douche, de savons pour les mains, de lotions pour le visage, de shampoings, d'après-shampoings, de produits pour le coiffage, de produits pour le bain, de lotions de démaquillage, de produits pour traiter la peau, notamment contre l'acné, de lotions pour améliorer le teint ou l'éclat de la peau, de lotions pour lutter contre le vieillissement, de lotions pour protéger la peau des agressions extérieures, notamment du soleil, de lotions pour le nettoyage de la cavité buccale, de lotions pour le nettoyage des parties génitales.

L'utilisation combinée des sophorolipides et d'au moins un solvant non polaire non aqueux permet aussi d'améliorer le mouillage des surfaces apolaires, améliore la qualité des jets de pulvérisation et améliore la pénétration foliaire, ce qui les rend efficaces pour le traitement des plantes.

Les compositions décrites seront donc aussi utilisées dans le domaine des traitements phytosanitaires, notamment pour le traitement des plantes ou leurs destructions, pour lutter contre les insectes ravageurs, pour lutter contre la prolifération des champignons et moisissures, pour lutter contre les gastéropodes, pour lutter contre la prolifération des mousses, des algues ou des lichens.

Dans ce domaine, les compositions décrites pourront contenir une ou plusieurs matières actives. On parle alors de formules "Ready-Mix" ou prêtes à l'emploi. Les compositions peuvent être absentes de matières actives, mais avoir des fonctionnalités complémentaires, notamment mouillantes, pénétrantes, anti-dérives, moussantes, anti-moussantes, compléxantes des ions notamment du calcium et du magnésium, d'ajustement du pH, colorantes. On parle alors de formules "Tank-mix" ou d'adjuvants extemporanés, le produit étant ajouté avant l'application en complément d'un autre produit contenant une matière active.

**EXEMPLES** :

Dans l'ensemble des exemples ci-dessous, les sophorolipides utilisés proviennent de la société WHEATOLEO sous le nom commercial "SOPHOCLEAN" et/ou de la société SOLIANCE sous le nom commercial "SOPHOLIANCE S". Ils sont issus d'un processus de fermentation utilisant *Candida bombicola* permettant la métabolisation du glucose et des esters méthyliques de colza en sophorolipides. Les produits commerciaux contiennent au moins 70% massique de sophorolipides par rapport à la masse sèche, le reste étant constitué principalement de sucres et d'acides gras. Les sophorolipides des deux références commerciales sont définis par les structures (1) et (2) décrites précédemment.

La forme majoritaire est une forme lactone, forme (1), en C18 :1, c'est-à-dire que la partie hydrophobe comporte 18 carbones et une insaturation et a subi une estérification intramoléculaire. Selon (1), R2 est un groupement méthyle, R1 est un alkyle linéaire avec 15 atomes de carbone et une insaturation. R3 et R4 sont des groupements acétyles. Cette forme représente de 15 à 40% de l'ensemble de la masse sèche.

La deuxième forme, représentant 10 à 40% de la masse sèche est une forme en C18 :1 ouverte, c'est-à-dire que la partie apolaire est constituée du même reste d'acide gras que précédemment, mais qu'il n'a pas subi d'estérification. Selon (2), R2' est un groupement méthyle, R1' est un alkyle linéaire avec 15 atomes de carbone et une insaturation, R3' et R4' sont des groupements acétyles et R5 est un groupement OCH3.

La troisième forme représentant 5 à 25% de la masse sèche est une forme ouverte en C18 :1. Selon (2), R2' est un groupement méthyle, R1' est un alkyle linéaire avec 15 atomes de carbone et une insaturation, R3' et R4' sont des groupements acétyles et R5 est un groupement OH.

La quatrième forme représentant 5 à 25% de la masse sèche est une forme en C18 :2 ouverte. Selon (2), R2' est un hydrogène, R1' est un alkyle linéaire avec 16 atomes de carbone et deux insaturations, R3' et R4' sont des groupements acétyles et R5 est un groupement OCH3.

La cinquième forme représentant 0 à 10% de la masse sèche est une forme lactone en C18 :2. Selon (1), R2 est un groupement méthyle, R1 est un alkyle linéaire avec 15 atomes de carbone et deux insaturations et R3 et R4 sont des groupements acétyles.

Les principales impuretés sont l'acide oléique, représentant moins de 20% de la masse sèche, et l'acide linoléique, représentant moins de 3% de la masse sèche.

Dans la suite les termes Sophorolipides, SOPHOLIANCE S, SOPHOCLEAN définissent tous les produits commerciaux tels que définis précédemment.

**1. Mise en évidence du pouvoir solubilisant**

**1.1 Solubilisation de colorants insolubles dans l'eau**

Les essais sont réalisés à l'aide de deux colorants insolubles, le Rouge de Soudan 7B (MM = 379.46 g/mol) commercialisé par Acros Chemical, et le Jaune au gras W1201 (MM=273.28 g/mol) commercialisé par la société LCW. Ces deux molécules sont des colorants synthétiques comportant des noyaux aromatiques et des fonctions azotées.

Le principe de la méthode est de mettre en contact une solution aqueuse de sophorolipides de concentration donnée (ici de 0,1 à 100g/l) avec un excès de pigment sous forme solide (un à deux bouts de spatules). La solution est laissée sous agitation à l'aide d'un barreau aimanté et d'une plaque d'agitation à la température du laboratoire (23 +/- 3°C) pendant 3 heures, puis filtrée à l'aide d'un filtre mono-usage d'acétate de cellulose d'un seuil de coupure de 0,45 $\mu$m. Le filtrat composé de la solution de sophorolipides et du pigment solubilisé est récupéré et le taux de pigment en mg/l mesuré à l'aide d'un spectromètre absorbant dans l'U.V et le visible (Hitachi U29000). La mesure est effectuée à la longueur d'onde d'absorption maximum du pigment donné, la ligne de base et la solution de référence étant une solution de sophorolipides ne contenant pas le pigment. L'appareil est étalonné dans les mêmes conditions par des solutions de pigments dans de l'éthanol.

Le tableau suivant donne les résultats obtenus exprimés en mg/l de pigment solubilisé par g/L de matière sèche de sophorolipides :

| Concentration de SOPHOLIANCE S | Jaune au gras solubilisé à pH =6 | Jaune au gras solubilisé à pH=8 | Jaune au gras solubilisé à pH=10 |
|---|---|---|---|
| 0,1 g/l | 0,16 mg/l | 0,36 mg/l | |
| 1 g/l | 0,33 mg/l | 0,59 mg/l | 1,14 mg/l |
| 10 g/l | 3,54 mg/l | 2,01 mg/l | 2,26 mg/l |
| 100 g/l | 95,24 mg/l | 75,69 mg/l | 66,77 mg/l |

| Concentration de SOPHOLIANCE S | Rouge Soudan 7B solubilisé à pH =6 | Rouge Soudan 7B solubilisé à pH=8 | Rouge Soudan 7B solubilisé à pH=10 |
|---|---|---|---|
| 0,1 g/l | 1,91 mg/l | | |
| 1 g/l | 7,30 mg/l | 1,85 mg/l | 3,2 mg/l |
| 10 g/l | 51,66 mg/l | 9,88 mg/l | 7,83 mg/l |
| 100 g/l | 632,05 mg/l | 316,11 mg/l | 256,98 mg/l |

**1.2 Solubilisation de parfums et huiles essentielles**

Cet exemple souhaite mettre en évidence la capacité de solubilisation des sophorolipides vis-à-vis des matières parfumées, notamment des parfums de synthèses et des huiles essentielles. La méthode consiste à rechercher la masse minimale en sophorolipides nécessaire pour solubiliser un gramme de composé insoluble dans l'eau. En pratique, on recherche la quantité minimale de sophorolipides nécessaire pour solubiliser 200 $\mu$l de parfums et/ou huiles complété à 5 g avec de l'eau. La masse minimale est trouvée par dichotomie, en observant les solutions misent au repos à la température du laboratoire (23 +/- 2°C). On cherche à obtenir des solutions parfaitement limpides, dans lesquelles la substance est complètement solubilisée.

Le tableau suivant donne les résultats obtenus exprimés en g de matière sèche de sophorolipides nécessaire pour solubiliser 1 g de matière non soluble dans l'eau.

| Huile essentielle de | SOPHOLIANCE S à pH=6 | SOPHOLIANCE S à pH=8 |
|---|---|---|
| Pamplemousse | 3,11 | 15,85 |
| Thym | 9,02 | 3,24 |
| Bergamote | 7,46 | 6,35 |

(suite)

| Huile essentielle de | SOPHOLIANCE S à pH=6 | SOPHOLIANCE S à pH=8 |
|---|---|---|
| Romarin | 2,47 | 7,49 |
| Menthe | 19,37 | 5,36 |
| Lavande | 8,05 | 5,38 |

### 1.3 Solubilisation d'actifs cosmétiques

La méthode de l'exemple 1.2 est reproduite avec des matières actives cosmétiques non solubles.

| Matière active | SOPHOLIANCE S à pH=6 | SOPHOLIANCE S à pH=8 |
|---|---|---|
| Eugènol | 22,22 | 4,78 |
| $\alpha$-Tocopherol | 6,52 | 4,47 |
| Lactate de menthyle | 17,72 | |

### 1.4 Exemples comparatifs

La capacité de solubilisation des sophorolipides est comparée à celle du Cremophore CO40 de la société BASF. Le nom INCI de ce dernier est PEG 40 Hydrogenated castor oil, qui est considéré comme une référence dans le domaine des solubilisants.

Pour l'huile essentielle de thym, la capacité de solubilisation du Chremophor CO40 est mesurée égale à 13,15 g de matière sèche pour solubiliser 1 g d'huile, résultat qu'il faut comparer à 3,24 g pour 1 g d'huile dans le cas de sophorolipides à pH égal à 8 et à 9,02 g pour 1 g à pH=6.

Pour l'huile essentielle de menthe, le résultat pour le Cremophor CO40 est de 16,14 g pour solubiliser 1 g d'huile, qu'il faut comparer à 5,36 g pour 1 g si on utilise les sophorolipides à pH=8 et à 19,37 g pour 1 g à pH=6.

Pour l'$\alpha$-Tocopherol, une des formes de la vitamine E, la capacité de solubilisation du Cremophor CO40 est de 6 g pour 0,2 g d'actif (donnée issue de la documentation technique de BASF). Celle des sophorolipides à pH=8 est de 0,9 g pour 0,2 g d'actif et de 1,3 g pour 0,2 g d'actif à pH=6.

## 2. Exemples de compositions (non revendiquées)

Les compositions suivantes donnent des solutions limpides et stables plusieurs mois à 4°C, 20°C et 35°C. Les pourcentages sont exprimés en masse de produit pour 100 g de composition.

**Composition 2.1 :** Solubilisation d'un Ester méthylique de Colza (insoluble)

| | |
|---|---|
| SOPHOCLEAN (MS=52%) | 90.4% |
| Ester méthylique de Colza (Radia 7961, Oleon) | 7% |
| Eau | 2.6% |
| MS : Matière Sèche | |

Compte tenu de la quantité d'eau présente dans SOPHOCLEAN, le pourcentage d'eau présent dans la composition est de 46% en poids.

Cette composition est diluable, c'est-à-dire que l'ajout d'eau supplémentaire garde la solution limpide et stable.

**Composition 2.2 :** Solubilisation de RhodiaSolv IRIS (solubilité dans l'eau = 2,5% en poids selon la documentation technique de la société Rhodia)

| | |
|---|---|
| SOPHOCLEAN (MS=52%) | 67.3% |
| Rhodiasolv IRIS (Rhodia) | 13% |
| Eau | 19.7% |

Compte tenu de la quantité d'eau présente dans SOPHOCLEAN, le pourcentage d'eau présent dans la composition est de 52% en poids.
Cette composition est diluable dans l'eau.

**Composition 2.3 :** formulation anti graffiti.

| | |
|---|---|
| SOPHOCLEAN (MS=54.6%) | 50% |
| Rhodiasolv IRIS (Rhodia) | 31% |
| Serdet DSK 40 (2 éthyl-hexyl-sulfate de sodium, 40%, Elementis) | 17% |
| Acide Glycolique (67%, Across Organinc) | 2% |

Compte tenu de la quantité d'eau présente dans Sophoclean et dans Serdet DSK40, le pourcentage d'eau présent dans la composition est de 32.9% en poids.
Des essais de nettoyage réalisés sur de la peinture sur matériaux poreux (parpaings) montrent une bonne efficacité de la préparation comme anti-graffiti utilisée pure ou diluée.

**Composition 2.4** : Solubilisation du Dertol 90 (terpinéol, insoluble)

| | |
|---|---|
| SOPHOCLEAN (MS=52%) | 88.5% |
| DERTOL 90 (DRT) | 6.9% |
| Eau | 3.5% |

Compte tenu de la quantité d'eau présente dans SOPHOCLEAN, le pourcentage d'eau présent dans la composition est de 46% en poids.
Cette composition est diluable.

**Composition 2.5 :** Solubilisation du succinate de DI-éthyle

| | |
|---|---|
| SOPHOCLEAN (MS=52%) | 84.6% |
| D.E.S (Bioamber) | 12% |
| Eau | 3.4% |

Compte tenu de la quantité d'eau présente dans SOPHOCLEAN, le pourcentage d'eau présent dans la composition est de 44% en poids.
Cette composition est diluable.

**Composition 2.6 :** Formulation d'un dégraissant concentré

| | |
|---|---|
| SOPHOCLEAN (MS=52%) | 53.3% |
| D.E.S (Bioamber) | 30% |
| Appyclean 6505 (MS=60%, Xyloside d'amyle, Wheatoleo) | 16.7% |

Compte tenu de la quantité d'eau présente dans SOPHOCLEAN et Appyclean 6505, le pourcentage d'eau présent dans la composition est de 32.3% en poids.

**Composition 2.7 :** Solubilisation de Rhodiasolve RPDE (solubilité = 5.7% en poids selon la documentation technique de la société Rhodia)

| | |
|---|---|
| SOPHOCLEAN (MS=52%) | 76,9% |
| Rhodiasolv RPDE (Rhodia) | 20% |
| Eau | 3,1% |

Compte tenu de la quantité d'eau présente dans SOPHOCLEAN, le pourcentage d'eau présent dans la composition est de 40% en poids.

**Composition 2.8 :** Solubilisation de Rhodiasolve RPDE (solubilité = 5.7% en poids selon la documentation technique de la société Rhodia)

| | |
|---|---|
| SOPHOCLEAN (MS=52%) | 44.2% |
| Rhodiasolv RPDE (Rhodia) | 43% |
| Appyclean 6505 (MS=60%, Xyloside d'amyle, Wheatoleo) | 12.8% |

Compte tenu de la quantité d'eau présente dans SOPHOCLEAN et Appyclean 6505, le pourcentage d'eau présent dans la composition est de 26.3% en poids.

**Exemple comparatif :** lotion pour le traitement de l'acné : Solubilisation de l'acide salicylique

| | Composition 2.9 | Composition comparative 2 |
|---|---|---|
| SOPHOLIANCE S (MS=55%) | 8.0% | 0% |
| Acide Salicylique | 0,5% | 0,5 % |
| Appyclean 6505 (MS=60%, Xyloside d'amyle, Wheatoleo) | 8.0% | 8,0% |
| Glycérol | 5.0% | 5,0% |
| Acide Citrique (20%) | Qsp pH=4.5 | Qsp pH=4,5 |
| Eau | QSp 100% | QSP 100% |
| Aspect après 24H au réfrigérateur (4°C) | Solution limpide | Solution trouble avec cristaux |

**Composition 2.10 :** Formulation d'un produit de lutte contre les mousses pour toiture : Solubilisation de l'acide pelargonique (acide nonanoïque).

| | Composition 2.10 |
|---|---|
| SOPHOCLEAN (MS=52%) | 19.2% |
| Acide nonanoïque (Aldrich) | 30.0% |
| Appyclean 6505 (MS=60%, Xyloside d'amyle, Wheatoleo) | 50.8% |

Compte tenu de la quantité d'eau présente dans SOPHOCLEAN et Appyclean 6505, le pourcentage d'eau présent dans la composition est de 29.54%.

**Composition 2.11 et 2.12:** Formulation d'un dégraissant ménager prêt-à-l'emploi

| | Composition 2.11 |
|---|---|
| SOPHOCLEAN (MS=54,5%) | 0,9 % |
| SERDET DSK 40 (40%, 2 éthyl-hexyl sulfonate de sodium, Elementis) | 3,3 % |
| Appyclean 6781 (60%, C8/C10 Wheat bran polyglycosides, Wheatoleo) | 0,8 % |
| Appyclean 6552 (60%, Amyl Xylosides & C10/C12 xylosides, Wheatoleo) | 0,8 % |
| Rhodiasolv RPDE (Rhodia) | 1,0 % |
| Metasilicate de sodium (30%) | 0,1% |
| Parfums Bambou | 0,2% |
| Eau | Qsp 100% |

Exemple Comparatif

| | Composition 2.12 | Composition comparative 3 |
|---|---|---|
| SOPHOCLEAN (54,5%) | 0,9 % | 0,9 % |
| SERDET DSK 40 (40%, 2 éthyl-hexyl sulfonate de sodium, Elementis) | 1,3 % | 1,3% |
| Rhodiasolv RPDE (Rhodia) | 1,0 % | 0% |
| Metasilicate de sodium (30%) | Qs pH=6 | Qs pH=6 |
| Parfums Bambou | 0,2% | 0,2% |
| Eau | QSp 100% | QSp 100% |

**3. Mise en évidence des actions renforcées.**

**3.1 Tension de surface dynamique**

La réduction dynamique des tensions de surface ou tension de surface dynamique est notamment décrite par Milton J. Rosen dans "Surfactants and interfacial phenomena", third edition, Wiley-Intersicence.

Les tensions de surface dynamiques sont des éléments importants à considérer dans de nombreux domaines industriels, et notamment pour les traitements phytosanitaires. Le mouillage des plantes aux surfaces épicuticullaires hydrophobes sera favorisé par des tensions de surfaces réduites dès les premières millisecondes de formation de l'interface. Les tensions de surface dynamiques sont mesurées à l'aide d'un tensiomètre à pression maximale de bulle mesurant la pression et le débit de bulle d'air sortant d'un capillaire de verre hydrophobe.

La relation entre la pression maximale Pmax, la pression hydrostatique dans le capillaire P0, le diamètre intérieur du tube r et la tension de surface $\gamma$ est exprimée selon l'équation 2.

$$\gamma = \frac{(P_{max} - P_0).r}{2} \qquad \text{eq.2}$$

Ici l'appareil utilisé est de marque KRUSS de type BP2 et les mesures sont réalisées à 25°C.

Les mesures sont réalisées sur des sophorolipides à 1 et 0,1% MS à pH=6 ainsi que sur des solutions de la composition 2.4 diluée 46 fois et 460 fois (correspondant à 1% de sophoroses lipides et 0,15% de Dertol 90 d'une part et à 0,1% de sophorolipides et 0,015% de Dertol 90 d'autre part).

| Age de l'interface (ms) | Tension de surface (mN/m) pour 1% Sophorolipides à pH=6 | Tension de surface (mN/m) pour la composition 2.4 diluée 46 fois (pH=6) |
|---|---|---|
| 10 | 48.9 | 42.2 |
| 100 | 42.1 | 38.7 |
| 500 | 39.2 | 36.5 |
| 1000 | 38.3 | 35.7 |

| Age de l'interface (ms) | Tension de surface (mN/m) pour 0,1% Sophorolipides à pH=6 | Tension de surface (mN/m) pour la composition 2.4 diluée 460 fois (pH=6) |
|---|---|---|
| 10 | 66.3 | 62.6 |
| 100 | 54 | 51.1 |
| 500 | 44 | 42.9 |
| 1000 | 41.3 | 40.4 |

Les résultats démontrent l'effet remarquable des compositions décrites sur l'abaissement des tensions de surfaces en fonction de la durée de création de l'interface air/liquide.

**3.2 Nettoyage des surfaces dures.**

La méthode choisie est issue de la méthode IKW, association allemande des industriels de la cosmétique, des soins et des détergents, pour juger de la performance des nettoyants touts usages et disponible à l'adresse : http://www.ikw.org/pdf/broschueren/EQ Allzweck englisch.pdf. Les tests sont réalisés à l'aide d'un abrasimètre humide de marque SHEEN de type 903 P&G. Des plaques de carrelage en céramique blanche de dimension 25x40 cm sont salies en déposant, à l'aide d'un aérographe, 0,6 à 0,9 g d'une salissure sur une bande de 8 cm de large.

Après vieillissement de 24 H dans une étuve ventilée à 100°C et 24 H à la température ambiante, les plaques sont nettoyées à l'aide de l'abrasimètre et de la solution de référence recommandée par IKW. 5 ml de solution serve à imprégner des tissus de nettoyage (de marque "la ménagère") disposés sur les supports spécifiques de l'appareil. L'abrasimètre réalise des allers-retours sur la plaque de céramique à la vitesse de 20 A/R par minute. La solution de référence sert à calibrer le nombre d'allers-retours et à noter l'effet de nettoyage, de 0 pour une plaque non nettoyée à 10 pour une plaque parfaitement propre. Les essais réalisés par la suite avec les solutions à tester sont effectués à un nombre d'aller-retours correspondant à la note 2 pour la solution de référence IKW.

La salissure a la composition suivante exprimée en % poids/poids :

| | |
|---|---|
| Huile d'arachide (alimentaire, marque supermarché) | 74,8 % |
| Kaolin Monohydrate (Acros Organics) | 23% |
| Noir de carbone (Acétylène, 50%, Alfa Aesar) | 2% |
| Agent de réticulation (Cobalt Naphthénate de cobalt, 6%, Aldrich) : | 0,2% |

**Exemple 3.1 : Résultats du test de nettoyage des surfaces dures :**

| | Note moyenne sur un nombre d'essais >5 (0 à 10) | Ecart type |
|---|---|---|
| Solution de Référence IKW | 2 | |
| SOPHOCLEAN 5% MS dans (1) | 6,2 | 0,8 |
| 10,4 % de la composition 2.1 dans (1), correspondant à 5% de SOPHOCLEAN + 0,75% d'ester méthylique de colza | 7 | 1,4 |
| 12,5% de la composition 2.7 dans (1) correspondant à 5% de SOPHOCLEAN + 2,5% Rhodiasolv RPDE | 8,1 | 1,9 |
| Exemple comparatif 4 (C12/14 Polyglucoside, GLUCOPON 600 de BASF, 5% MS dans (1)) | 4 | 0,7 |
| (1) est une solution de $Na_2HPO_4$ à 2,5% tamponnant le pH à 7,5. | | |

Les résultats montent l'efficacité de SOPHOCLEAN, par rapport à l'exemple comparatif 4, et l'effet remarquable des compositions décrites sur le résultat de nettoyage des surfaces dures.

**Exemple 3.2 : Résultats des tests de nettoyage des surfaces dures**

| | Note moyenne après 40 Allers-retours |
|---|---|
| Composition 2.12 (avec solvant) | 6 |
| Composition comparative 3 (sans solvant) | 3,5 |

Ces résultats démontrent de l'effet remarquable des compositions décrites sur le nettoyage des surfaces dures.

L'invention vise aussi l'utilisation d'au moins un sophorolipide comme solubilisant, dans laquelle de préférence la forme (1) représente de 5 à 65%, de préférence de 10 à 50% de la masse totale des sophorolipides.

**3.3 Efficacité des préparations pour lutter contre les mauvaises herbes.**

La préparation de l'exemple 2.4, contenant des alcools terpéniques solubilisés par des sophorolipides, est utilisée comme adjuvant extemporané du glyphosate. Le glyphosate est un herbicide non sélectif, dont la définition et l'usage sont notamment décrits dans « The biochemistry and uses of pesticides, 2nd edition », de Kenneth A. Hassall aux éditions VCH (1990) . Un adjuvant extemporané est une préparation ajoutée à la préparation phytosanitaire au moment de l'application et visant à renforcer l'action de cette dernière. Un adjuvant extemporané ou « tank-mix » ne contient pas lui même de molécules biologiquement actives.

Le glyphosate utilisé est une solution de glyphosate sous la forme de sel d'isopropylamine titrée à 620 g/l de glyphosate équivalent acide.

L'efficacité de la préparation décrite est jugée en réalisant des courbes d'efficacités biologiques, c'est-à-dire en déterminant la capacité des préparations à réduire la biomasse d'une plante cible en fonction de la concentration en herbicide. Cette méthode d'évaluation est notamment décrite dans « Herbicide Bioassays » de Jens C. Streibig et Per Kudsk, aux éditions CRC press (1993).

L'évolution de la biomasse avec la concentration en herbicide suit l'équation suivante :

$$Y=C + [(D-C)/(1+exp(-2(a+b\ log(R.X)))]$$

où Y est la biomasse sèche de la plante (plante coupée à la base et séchée 24H à 110°C dans une étuve ventilée), X est la concentration en herbicide exprimée en gramme de glyphosate acide par hectare. C, D, a et b étant des facteurs

déterminés par l'analyse de la courbe selon la méthode des moindres carrés. R représente le facteur d'efficacité, et est égal à 1 pour le traitement à l'herbicide sans adjuvant, sera >1 si l'adjuvant renforce l'efficacité de l'herbicide et sera <1 en cas de perte d'efficacité. Sa valeur est déterminée en appliquant l'équation précédente avec les paramètres D,C,a et b de la courbe de l'herbicide sans adjuvant (R=1), selon la méthode des moindres carrés.

La plante testée est ici *Avena Fatua* dans des pots de 10 cm de diamètre (4 plantes par pot, et 4 répétitions). Le traitement est réalisé 3 semaines après le semis au stade 2 feuilles, à l'aide d'un pulvérisateur muni d'une buse pinceau permettant de délivrer à 2,4 bar 200L par hectare. Les plantes sont laissées dans une chambre phytotronique permettant de fixer les conditions climatiques suivantes :

- température de 23°C/18°C jour/nuit
- 70% d'humidité relative
- Photopériode de 16h (nuit de 1h à 9h du matin)
- Intensité lumineuse de 158$\mu$mol.m$^{-2}$.s$^{-1}$

Les plantes sont coupées et séchées 3 semaines après le traitement.

L'adjuvant selon la composition décrite est ajouté à l'herbicide juste avant le traitement à la dose de 400g/ha quelle que soit la concentration en herbicide (variant de 50 à 650 g équivalent acide par hectare).

Le résultat est exprimé selon la valeur de R selon l'équation précédente.

| | Glyphosate (sel d'isopropylamine) | Glyphosate (sel d'isopropylamine) + Composition de l'exemple 2,4 à 400L/ha |
|---|---|---|
| R | 1 | 4,66 |

L'analyse statistique selon Anova (p<0,05) montre que la différence entre les deux valeurs de R est hautement significative, et démontre le gain d'efficacité obtenue grâce à la composition 2,4 décrite.

## Revendications

1. Utilisation d'au moins un sophorolipide comme solubilisant.

2. Utilisation suivant la revendication 1, dans laquelle le sophorolipide est de structure (1) et/ou (2)

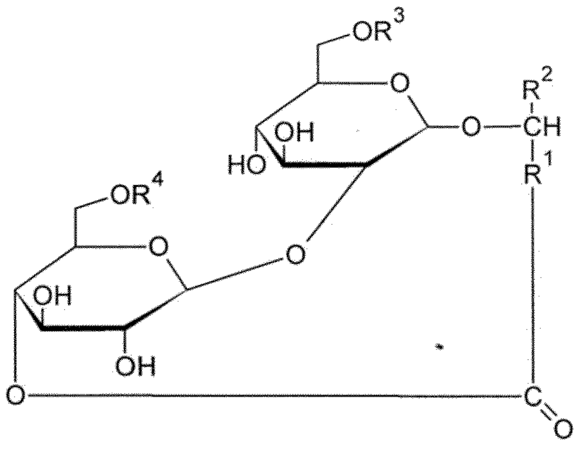

(1)

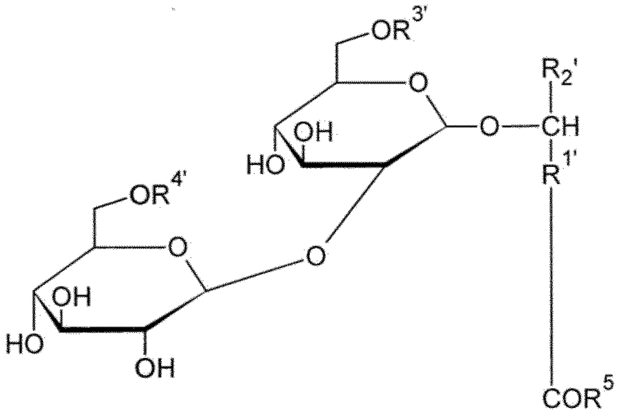

(2)

- R1 et R1' sont indépendamment les unes des autres des chaînes hydrocarbonées saturées ou ayant une ou plusieurs insaturations, non hydroxylées ou possédant un ou plusieurs groupements hydroxyles, linéaires ou ramifiées ayant 1 à 21 atomes de carbone,
- R2 et R2' sont indépendamment les uns des autres un atome d'hydrogène ou un radical alkyle saturé ou ayant une ou plusieurs insaturations, non hydroxylé ou possédant un ou plusieurs groupements hydroxyles, linéaire ou ramifié ayant 1 à 9 atomes de carbone,
- R3, R4, R3' et R4' sont indépendamment les uns des autres un atome d'hydrogène ou un groupement acétyle, et
- R5 est un groupement OCH3 ou OH ou $O^-M^+$ où M+ est un ion métallique ou un cation organique notamment des sels d'ammonium comme de diméthylammonium, triméthylammonium, isopropylammonium, mono-éthanol-ammonium, di-éthanol-ammonium, tri-éthanol-ammonium, dis phosphonium ou $O(CH2)_mCH3$ avec m compris entre 1 et 11.

3. Utilisation suivant la revendication 2, dans laquelle la forme (1) représente de 5 à 65%, de préférence de 10 à 50% de la masse totale des sophorolipides.

4. Utilisation suivant l'une quelconque des revendications 1 à 3, **caractérisée en ce que** les sophorolipides sont issus directement du processus de fermentation ou extraits du milieu de fermentation.

5. Utilisation suivant l'une quelconque des revendications 1 à 4 pour obtenir une solution limpide.

6. Utilisation suivant l'une quelconque des revendications 1 à 5 pour solubiliser au moins une matière non soluble dans l'eau.

7. Utilisation suivant la revendication 6, **caractérisée en ce que** la matière non soluble est un solvant non polaire ayant un paramètre polaire de Hansen inférieur à 5,5 $MPa^{1/2}$.

8. Utilisation suivant la revendication 6, **caractérisée en ce que** la matière non soluble est un parfum ou une huile essentielle.

9. Utilisation suivant la revendication 6, **caractérisée en ce que** la matière non soluble est un actif cosmétique, pharmaceutique ou phytopharmaceutique.

10. Utilisation suivant la revendication 6, **caractérisée en ce que** le rapport de masse entre le solubilisant et la matière solubilisée varie de 1 à 9000 et de préférence de 1,6 à 90, et l'eau compose de 1 à 99,89 % de la masse totale de la matière solubilisée, du solubilisant et de l'eau.

**Patentansprüche**

1. Verwendung mindestens eines Sophorolipids als Löslichkeitsvermittler.

2. Verwendung gemäß Anspruch 1, wobei das Sophorolipid der Struktur (1) und/oder (2) entspricht

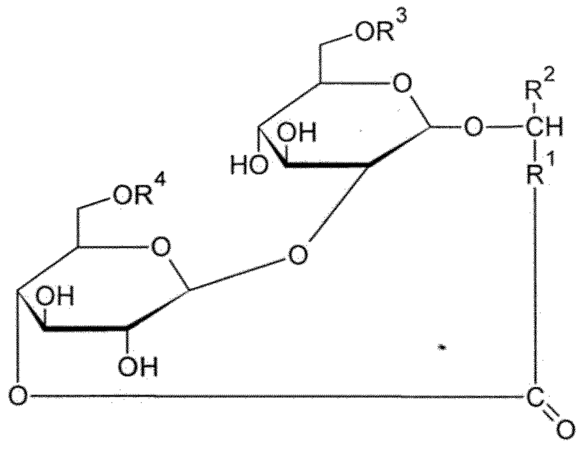

(1)

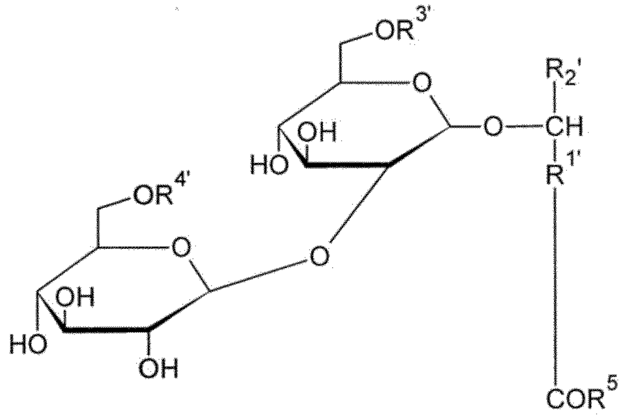

(2)

- R1 und R1' unabhängig voneinander für Kohlenwasserstoffketten stehen, die gesättigt sind oder die eine oder mehrere ungesättigte Stellen aufweisen, wobei sie hydroxyliert sind oder eine oder mehrere Hydroxylgruppen besitzen, von geradkettiger oder verzweigter Art mit 1 bis 21 Kohlenstoffatomen,
- R2 und R2' unabhängig voneinander für ein Wasserstoffatom oder für einen Alkylrest stehen, der gesättigt ist oder der eine oder mehrere ungesättigte Stellen aufweist, wobei er hydroxyliert ist oder eine oder mehrere Hydroxylgruppen besitzt, von geradkettiger oder verzweigter Art mit 1 bis 9 Kohlenstoffatomen,
- R3, R4, R3' und R4' unabhängig voneinander für ein Wasserstoffatom oder eine Acetylgruppe stehen, und
- R5 eine Gruppe vom Typ $OCH_3$ oder OH oder $O^-M^+$ ist, wobei M+ ein Metallion oder ein organisches Kation ist, insbesondere von Ammoniumsalzen wie von Dimethylammonium, Trimethylammonium, Isopropylammonium, Monoethanolammonium, Diethanolammonium, Triethanolammonium, Bis-phosphonium, oder $O(CH_2)_mCH_3$, wobei m im Bereich von 1 und 11 liegt.

**3.** Verwendung gemäß Anspruch 2, wobei die Form (1) 5 bis 65%, vorzugsweise 10 bis 50%, der Gesamtmasse der Sophorolipide ausmacht.

**4.** Verwendung gemäß einem beliebigen der Ansprüche 1 bis 3, **dadurch gekennzeichnet, dass** die Sophorolipide unmittelbar aus dem Fermentationsvorgang stammen oder Extrakte aus dem Fermentationsmedium sind.

**5.** Verwendung gemäß einem beliebigen der Ansprüche 1 bis 4, um eine klare Lösung zu erhalten.

**6.** Verwendung gemäß einem beliebigen der Ansprüche 1 bis 5, um mindestens einen unlöslichen Stoff in Wasser zu solubilisieren.

**7.** Verwendung gemäß Anspruch 6, **dadurch gekennzeichnet, dass** es sich bei dem unlöslichen Stoff um ein unpolares Lösungsmittel handelt, das einen polaren Hansen-Parameter von weniger als 5,5 $MPa^{1/2}$ hat.

**8.** Verwendung gemäß Anspruch 6, **dadurch gekennzeichnet, dass** es sich bei dem unlöslichen Stoff um einen

Duftstoff oder ein ätherisches Öl handelt.

9. Verwendung gemäß Anspruch 6, **dadurch gekennzeichnet, dass** es sich bei dem unlöslichen Stoff um einen kosmetischen, pharmazeutischen oder phytopharmazeutischen Wirkstoff handelt.

10. Verwendung gemäß Anspruch 6, **dadurch gekennzeichnet, dass** das Massenverhältnis zwischen dem Löslichkeitsvermittler und dem solubilisierten Stoff im Bereich von 1 bis 9.000 und vorzugsweise von 1.6 bis 90 liegt, wobei Wasser 1 bis 99,89% der Gesamtmasse aus solubilisiertem Stoff, Löslichkeitsvermittler und Wasser ausmacht.

## Claims

1. Use of at least one sophorolipid as solubilizer.

2. Use according to Claim 1, in which the sophorolipid is of structure (1) and/or (2)

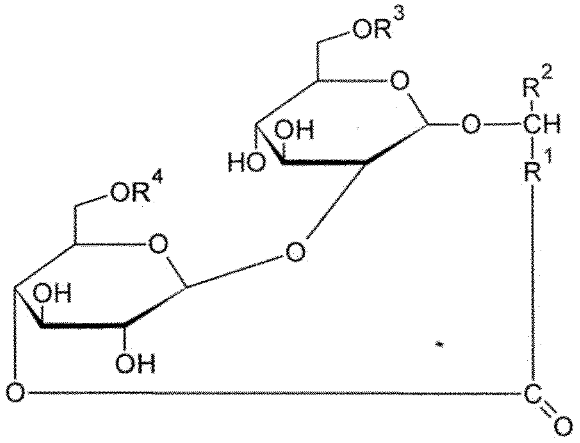

(1)

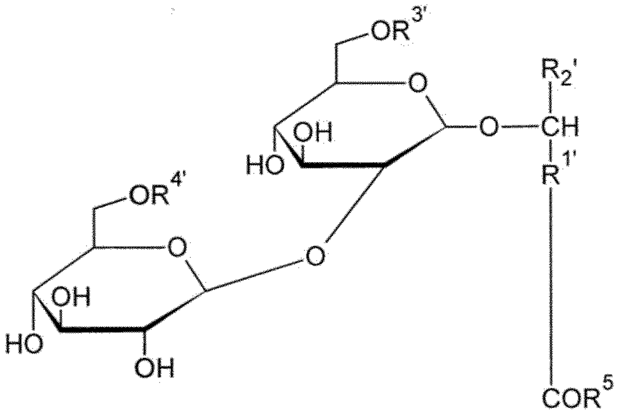

(2)

- $R^1$ and $R^{1'}$ are, independently of one another, linear or branched hydrocarbon chains having from 1 to 21 carbon atoms which are saturated or which have one or more unsaturations and which are non-hydroxylated or which have one or more hydroxyl groups,
- $R^2$ and $R^{2'}$ are, independently of one another, a hydrogen atom or a linear or branched alkyl radical having from 1 to 9 carbon atoms which is saturated or which has one or more unsaturations and which is non-hydroxylated or which has one or more hydroxyl groups,
- $R^3$, $R^4$, $R^{3'}$ and $R^{4'}$ are, independently of one another, a hydrogen atom or an acetyl group, and
- $R^5$ is an $OCH_3$ or OH or $O^-M^+$ group, where $M^+$ is a metal ion or an organic cation, in particular ammonium salts, such as salts of dimethylammonium, trimethylammonium, isopropylammonium, monoethanolammonium, diethanolammonium, triethanolammonium, bisphosphonium, or $O(CH_2)_mCH_3$, with m between 1 and 11.

3. Use according to Claim 2, in which the form (1) represents from 5% to 65%, preferably from 10% to 50%, of the total weight of the sophorolipids.

4. Use according to any one of Claims 1 to 3, **characterized in that** the sophorolipids result directly from the fermentation process or are extracts of the fermentation medium.

5. Use according to any one of Claims 1 to 4 for obtaining a clear solution.

6. Use according to any one of Claims 1 to 5 for dissolving at least one water-insoluble substance.

7. Use according to Claim 6, **characterized in that** the insoluble substance is a non-polar solvent having a Hansen polar parameter of less than 5.5 $MPa^{1/2}$.

8. Use according to Claim 6, **characterized in that** the insoluble substance is a fragrance or an essential oil.

9. Use according to Claim 6, **characterized in that** the insoluble substance is a cosmetic, pharmaceutical or phytopharmaceutical active substance.

10. Use according to Claim 6, **characterized in that** the weight ratio of the solubilizer to the dissolved substance varies from 1 to 9000 and preferably from 1.6 to 90 and the water makes up from 1% to 99.89% of the total weight of the dissolved substance, of the solubilizer and of the water.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006069175 A2
- WO 2005089522 A
- WO 2007130738 A
- WO 9701343 A
- WO 2004108063 A
- EP 0209783 A
- FR 2779057 A1
- US 6057302 A, BORZEIX FREDERIQUE
- US 5756471 A, HILLION GERARD
- US 6262038 B1, PIERCE DEBORAH
- JP 2009275145 A
- EP 0499434 B
- EP 1411111 A1
- EP 1445302 A2
- WO 2011051161 A1
- FR 2779057
- WO 2011051161 A
- US 6262038 B
- JP 2009275145 B


### Littérature non-brevet citée dans la description


- **MC BAIN.** *Journal of Chemical society,* 1918, vol. 113, 825
- *Canadian Journal of Chemistry,* 1961, vol. 39, 846
- **CHARLES M. HANSEN.** Hansen Solubility Parameters : A user's handbook. CRC Press
- **VAN KERVELEN.** Properties of Polymers. Elsevier, 1990
- The Pesticide Manual. British Crop Protection Counci
- **MILTON J. ROSEN.** Surfactants and interfacial phenomena. Wiley-Intersicence
- **KENNETH A. HASSALL.** The biochemistry and uses of pesticides. 1990
- **JENS C. STREIBIG ; PER KUDSK.** Herbicide Bioassays. 1993